# EUROPEAN PATENT APPLICATION

(11) **EP 2 128 142 A1**
(43) Date of publication of application: **02.12.2009**
(21) Application number: 08157288.5
(22) Date of filing: 30.05.2008
(51) Int. Cl.: C07D 251/46, C07D 251/52, C08G 65/26, C11D 1/72

(54) **Amphiphilic molecules with a triazine core**

(71) Applicant: BASF SE, 67056 Ludwigshafen (DE)
(72) Inventor: Garnier, Sebastien Dr., 69469 Weinheim (DE); Reinoso Garcia, Marta Dr., 68163 Mannheim (DE); Oetter, Günter Dr., 67227 Frankenthal (DE)

(57) **Abstract**

The present invention is directed to Chemical compound according to formula (I) 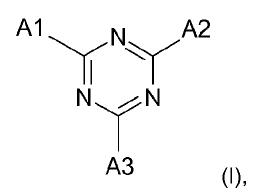 wherein
A1 and A2 independent from each other comprise a hydrophilic block (hi) and A3 comprises a hydrophobic block (ho) or wherein A1 and A2 independent from each other comprise a hydrophobic block (ho) and A3 comprises a hydrophilic block (hi).

## Description

The present invention is directed to a chemical compound according to formula (I) 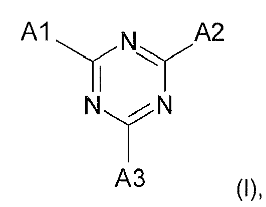 wherein
A1 and A2 independent from each other comprise a hydrophilic block (hi) and A3 comprises a hydrophobic block (ho) or wherein A1 and A2 independent from each other comprise a hydrophobic block (ho) and A3 comprises a hydrophilic block (hi).

Amphiphilic molecules with linear or branched alkyl chains are well known. Whilst some of them are performing well as emulsifiers, dispersing agents and solubilizers most of them do not perform well when used for aromatic hydrophobic actives and performance chemicals. For these purposes - there exists a need for amphiphilic molecules with new structures. These amphiphilic molecules should have an enhanced interaction between each other thus leading to an increase in micellar stability and by that to more stable emulsions, dispersions and interfacial layers.

This need has surprisingly been meet by the compound according to claims 1 to 5, the compositions according to claims 6 to 10 and the use according to claim 11.

The present invention is directed to a chemical compound according to formula (I) 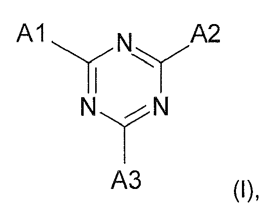 wherein
A1 and A2 independent from each other comprise a hydrophilic block (hi) and A3 comprises a hydrophobic block (ho) or wherein A1 and A2 independent from each other comprise a hydrophobic block (ho) and A3 comprises a hydrophilic block (hi).

A hydrophilic block (hi) is a structure, which can transiently bond with water through hydrogen bonding and consequently is soluble in water and other polar solvents.

A hydrophobic block (ho) is a structure, which is repelled from a mass of water and consequently is insoluble in water and polar solvents.

A chemical compound as described above is preferred, wherein hi is a non-ionic block and a compound is even more preferred, wherein the non-ionic block(s) is/are polyethyleneoxide.

Also for ho there exist preferred embodiments. A chemical compound according to the invention, wherein ho is selected from the group consisting of amines having 1 to 100 C-atoms is preferred. The amine preferably has 2 to 50, more preferred 3 to 18, even more preferred 4 to 12 and most preferred 5 to 8 C-atoms. The amines can have one or two alkyl chains. Amines having one alkyl chain are preferred.

It is particularly preferred, if the compounds according to the invention comprise one or two hi, which hi is a non-ionic block and even more preferred a polyethyleneoxide or polyethyleneglycol, and also comprise one or two ho, which ho is selected from the group consisting of amines having 1 to 100 C-atoms.

A chemical compound as described above, wherein A1 and A2 have the same structure is particularly preferred.

A chemical compound according to the present invention can be produced by reacting cyanuric chloride with e.g. an alcohol, which in addition to the OH-functionality also comprises ether-functionalities, such as polyethyleneoxide, and e.g. an amine or an alcohol, which has no additional functionalities besides the amine/alcohol-functionality, such as methylpentylamide. The substitution of the cyanuric chloride can be controlled e.g. by the reaction temperature.

A chemical composition comprising at least one inventive chemical compound is another object of the present invention.

There exist preferred amounts in which the inventive compound is present in the inventive chemical composition. Therefore a chemical composition, wherein the at least one inventive chemical compound is present in an amount of 0.001 to 99.9 mass%, preferably in an amount of 0.01 to 99.5 mass%, more preferred in an amount of 0.1 to 99 mass%, even more preferred in an amount of 1 to 95 mass% and most preferred in an amount of 5 to 50 mass% forms a preferred object of the present invention.

Such chemical composition preferably contains at least one compound selected from the group consisting of surfactant, disinfectant, dye, acid, base, complexing agent, biocide, hydrotope, thickener, builder, cobuilder, enzyme, bleaching agent, bleach activator, bleaching catalyst, corrosion inhibitor, dye protection additive, dye transfer inhibitor, anti-greying agent, soil-release-polymer, fiber protection agent, silicon, bactericide, preserving agent, organic solvent, solubility adjustor, solubility enhancer and perfume.

Surfactants normally consist of a hydrophobic and a hydrophilic part. Thereby the hydrophobic part normally has a chain length of 4 to 20 C-atoms, preferably 6 to 19 C-atoms and particularly preferred 8 to 18 C-atoms. The functional unit of the hydrophobic group is generally an OH-group, whereby the alcohol can be linear or branched. The hydrophilic part generally consists substantially of alkoxylated units (e.g. ethylene oxide (EO), propylene oxide (PO) and/or butylene oxide (BuO), whereby generally 2 to 30, preferably 5 to 20 of these alkoxylated units are annealed, and/or charged units such as sulfate, sulfonate, phosphate, carbonic acids, ammonium und ammonium oxide.

Examples of anionic surfactants are: carboxylates, sulfonates, sulfo fatty acid methylesters, sulfates, phosphates. Examples for cationic surfactants are: quartery ammonium compounds. Examples for betaine-surfactants are: alkyl betaines. Examples for non-ionic compounds are: alcohol alkoxylates.

A "carboxylate" is a compound, which comprises at least one carboxylate-group in the molecule. Examples of carboxylates, which can be used according to the present invention, are
➢ soaps - e.g. stearates, oleates, cocoates of alkali metals or of ammonium,
➢ ethercarboxylates - e.g. Akypo® RO 20, Akypo® RO 50, Akypo® RO 90.

A "sulfonate" is a compound, which comprises at least one sulfonate-group in the molecule. Examples of sulfonates, which can be used according to the invention, are
➢ alkyl benzene sulfonates - e.g. Lutensit® A-LBS, Lutensit® A-LBN, Lutensit® A-LBA, Marlon® AS3, Maranil® DBS,
➢ alkyl sulfonates - e.g. Alscoap OS-14P, BIO-TERGE® AS-40, BIO-TERGE® AS-40 CG, BIO-TERGE® AS-90 Beads, Calimulse® AOS-20, Calimulse® AOS-40, Calsoft® AOS-40, Colonial® AOS-40, Elfan® OS 46, Ifrapon® AOS 38, Ifrapon® AOS 38 P, Jeenate® AOS-40, Nikkol® OS-14, Norfox® ALPHA XL, POLYSTEP® A-18, Rhodacal® A-246L, Rhodacal® LSS-40/A,
➢ sulfonated oils such as Turkish red oil,
➢ olefine sulfonates,
➢ aromatic sulfonates - e.g. Nekal® BX, Dowfax® 2A1.

A "sulfo fatty acid methylester" is a compound, having the following general formula (II): 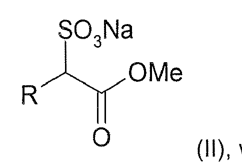 wherein R has 10 to 20 C-atoms; preferably 12 to 18 and particularly preferred 14 to 16 C-atoms.

A "sulfate" is a compound, which comprises at least one SO₄-group in the molecule. Examples of sulfates, which can be used according to the present invention, are
➢ fatty acid alcohol sulfates such as coco fatty alcohol sulfate (CAS 97375-27-4) - e.g. EMAL® 10G, Dispersogen® SI, Elfan® 280, Mackol® 100N,
➢ other alcohol sulfates - e.g. Emal® 71, Lanette® E,
➢ coco fatty alcohol ethersulfates - e.g. Emal® 20C, Latemul® E150, Sulfochem® ES-7, Texapon® ASV-70 Spec., Agnique SLES-229-F, Octosol 828, POLYSTEP® B-23, Unipol® 125-E, 130-E, Unipol® ES-40,
➢ other alcohol ethersulfates - e.g. Avanel® S-150, Avanel® S 150 CG, Avanel® S 150 CG N, Witcolate® D51-51, Witcolate® D51-53.

A "phosphate" is a compound, which comprises at least one PO₄-group. Examples of phosphates, which can be used according to the present invention, are
➢ alkyl ether phosphates - e.g. Maphos® 37P, Maphos® 54P, Maphos® 37T, Maphos® 210T and Maphos® 210P,
➢ phosphates such as Lutensit A-EP,
➢ alkyl phosphates.

When producing the chemical composition of the present invention the anionic surfactants are preferably added as salts. Acceptable salts are e.g. alkali metal salts, such as sodium-, potassium- and lithium salts, and ammonium salts, such as hydroxyl ethylammonium-, di(hydroxy-ethyl)ammonium- und tri(hydroxyethyl)ammonium salts.

One group of the cationic surfactants are the quartery ammonium compounds. A "quartery ammonium compound" is a compound, which comprises at least one R₄N⁺-group per molecule. Examples of counter ions, which are useful in the quartery ammonium compounds, are
➢ halogens, methosulfates, sulfates and carbonates of coco fat-, sebaceous fat- or cetyl/oleyltrimethylammonium.

Particularly suitable cationic surfactants are:
- N,N-dimethyl-N-(hydroxy-C₇-C₂₅-alkyl)ammonium salts;
- mono- and di-(C₇-C₂₅-alkyl)dimethylammonium compounds, which were quarternised with alkylating agents
- esterquats, especially mono-, di- and trialkanolamines, quarternary esterified by C₈-C₂₂-carbonic acids;
- imidazolinquats, especially 1-alkylimidazoliniumsalts of formula III or IV 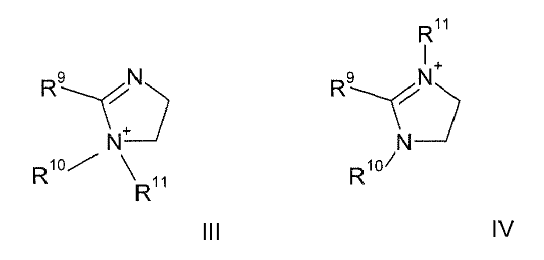
wherein the variables have the following meaning:
R⁹ C₁-C₂₅-alkyl or C₂-C₂₅-alkenyl;
R¹⁰ C₁-C₄-alkyl or hydroxy-C₁-C₄-alkyl;
R¹¹ C₁-C₄-alkyl, hydroxy-C₁-C₄-alkyl or a rest R¹-(CO)-X-(CH₂)ₘ- (X:-O- or -NH-; m: 2 or 3),
   whereby at least one rest R⁹ is C₇-C₂₂-alkyl.

A "betain-surfactant" is a compound, which comprises under conditions of use - i.e. in the case of textile washing under normal pressure and at temperatures of from room temperature to 95 °C - at least one positive charge and at least one negative charge. An "alkylbetain" is a betain-surfactant, which comprises at least one alkyl-unit per molecule. Examples of betain-surfactants, which can be used according to the invention, are Cocamidopropylbetain - e.g. MAFO® CAB, Amonyl® 380 BA, AMPHOSOL® CA, AMPHOSOL® CG, AMPHOSOL® CR, AMPHOSOL® HCG; AMPHOSOL® HCG-50, Chembetaine® C, Chembetaine® CGF, Chembetaine® CL, Dehyton® PK, Dehyton® PK 45, Emery® 6744, Empigen® BS/F, Empigen® BS/FA, Empigen® BS/P, Genagen® CAB, Lonzaine® C, Lonzaine® CO, Mirataine® BET-C-30, Mirataine® CB, Monateric® CAB, Naxaine® C, Naxaine® CO, Norfox® CAPB, Norfox® Coco Betaine, Ralufon® 414, TEGO®-Betain CKD, TEGO® Betain E KE 1, TEGO®-Betain F, TEGO®-Betain F 50 and aminoxides such as alkyl dimethyl amineoxide, i.e. compounds of general formula (V) 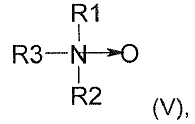 whereby R1, R2 and R3 are chosen independently from each other of an aliphatic, cyclic or tertiary alkyl- or amido alkyl-moiety, e.g. Mazox® LDA, Genaminox®, Aromox® 14 DW 970.

Non-ionic surfactants are interfacially active substances having a head group, which is an uncharged, polar, hydrophilic group, not carrying a ionic charge at neutral pH, and which head group makes the non-ionic surfactant water soluble. Such a surfactant adsorbs at interfaces and aggregates to micelles above the critical micelle concentration (cmc). According to the type of the hydrophilic head group it can be distinguished between (oligo)oxyalkylene-groups, especially (oligo)oxyethylene-groups, (polyethyleneglycol-groups), including fatty alcohol polyglycole ether (fatty alcohol alkoxylates), alkylphenol polyglycolether and fatty acid ethoxylates, alkoxylated triglycerides and mixed ethers (polyethylene glycolether alcoxylated on both sides); and carbohydrate-groups, including e.g. alkyl polyglucosides and fatty acid-*N*-methylglucamides.

Alcohol alkoxylates, are based on a hydrophobic part having a chain length of 4 to 20 C-atoms, preferably 6 to 19 C-atoms and particularly preferred 8 to 18 C-atoms, whereby the alcohol can be linear or branched, and a hydrophilic part, which can be alkoxylated units, e.g. ethylene oxide (EO), propylene oxide (PO) and/or butylene oxide (BuO), having 2 to 30 repeating units. Examples are besides others Lutensol ® XP, Lutensol ® XL, Lutensol ® ON, Lutensol ® AT, Lutensol ® A, Lutensol ® AO, Lutensol ® TO.

Alcoholphenolalkoxylates are compounds according to general formula (VI), 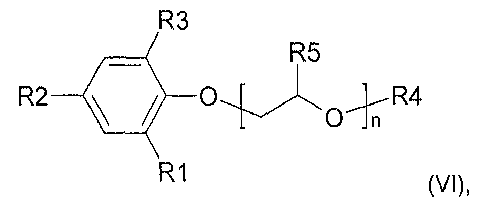 which can be produced by addition of alkylene oxide, preferably ethylene oxide onto alkyle phenoles. Preferably R4 = H. It is also preferred, if R5 = H, - since than it is EO; in the same way it is preferred if R5 = CH₃, since than it is PO, or, if R5 = CH₂CH₃ since than it is BuO. A compound is especially preferred, in which octyl- [(R1 = R3 = H, R2 = 1,1,3,3-tetramethylbutyl (diisobutylene)], nonyl- [(R1 = R3 = H, R2 = 1,3,5-trimethylhexyl (tripropylene)], dodecyl-, dinonyl- or tributylphenolpolyglycolether (e.g. EO, PO, BuO), R-C₆H₄-O-(EO/PO/BuO)n with R = C8 to C12 and n = 5 to 10, are present. Non-limiting examples of such compounds are: Norfox® OP-102, Surfonic® OP-120, T-Det® O-12.

Fatty acid ethoxilates are fatty acid esters, which have been treated with different amounts of ethylene oxide (EO).

Triglycerides are esters of the glycerols (glycerides), in which all three hydroxy-groups have been esterified using fatty acids. These can be modified by alkylene oxides.

Fatty acid alkanol amides are compounds of general formula (VII) 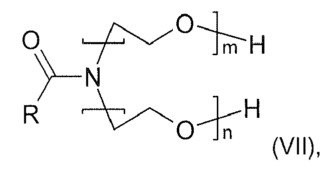 which comprise at least one amide-group having one alkyle moiety R and one or two alkoxyl-moiety(ies), whereby R comprises 11 to 17 C-atoms and 1 ≤ m + n ≤ 5.

Alkylpolyglycosides are mixtures of alkylmonoglucosides (alkyl- α-D- and - β-D-giucopyranoside plus small amounts of -glucofuranoside), alkyldiglucosides (-isomaltosides, -maltosides and others) and alkyloligoglucosides (-maltotriosides, -tetraosides and others). Alkylpolyglycosides are among other routes accessible by acid catalysed reaction (Fischer-reaction) from glucose (or starch) or from *n*-butylglucosides with fatty alcohols. Alkylpolyglycosides fit general formula (VIII) 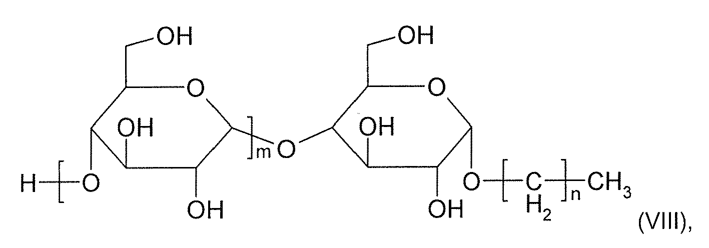 with
m = 0 to 3 and
n = 4 to 20.

One example is Lutensol ® GD70.

In the group of non-ionic *N*-alkylated, preferably *N*-methylated, fatty acid amides of general formula (IX) 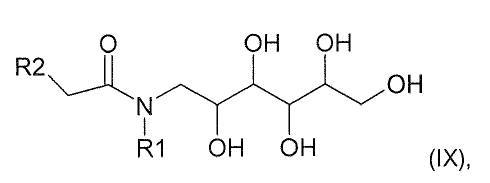 R1 is a *n*-C₁₂-alkyl-moiety, R2 an alkyl-moiety having 1 to 8 C-atoms. R2 preferably is methyl.

A composition as described, which additionally comprises a disinfectant is preferred. The at least one disinfectant preferably is present in an (overall) amount of 0.1 to 20 mass%, preferably 1 to 10 mass% of the composition.

Disinfectants can be: oxidation agents, halogens such as chlorine and iodine and substances, which release the same, alcohols such as ethanol, 1-propanol and 2-propanol, aldehydes, phenoles, ethylene oxide, chlorohexidine and mecetroniummetilsulfate.

The advantage of using disinfectants is that pathogenic germs can hardly grow. Pathogenic germs can be: bacteria, spores, fungi and viruses.

Dyes can be besides others: Acid Blue 9, Acid Yellow 3, Acid Yellow 23, Acid Yellow 73, Pigment Yellow 101, Acid Green 1, Acid Green 25.

A composition is preferred, in which the at least one dye is present in an (overall) amount of 0.1 to 20 mass%, preferably 1 to 10 mass%, of the composition.

Acids are compounds that can advantageously be used to solve or to avoid scaling. Non-limiting examples of acids are formic acid, acetic acid, citric acid, hydrochloric acid, sulfuric acid and sulfonic acid.

Bases are compounds, which are useful for adjusting a preferable pH-range for complexing agents. Examples of bases, which can be used according to the present invention, are: NaOH, KOH and amine ethanol.

As inorganic builder the following are especially useful:
- crystalline and amorphous alumo silicates having ion exchanging properties, such as zeolites: different types of zeolites are useful, especially those of type A, X, B, P, MAP and HS in their Na-modification or in modifications in which Na is partially substituted by other cat ions such as Li, K, Ca, Mg or ammonium;
- crystalline silicates, such as disilicates and layer-silicates, e.g. δ- and β-Na₂Si₂O₅. The silicates can be used as alkali metal-, earth alkali metal- or ammonium salts, the Na-, Li- and Mg-silicates are preferred;
- amorphous silicates, such as sodium metasilicate and amorphous disilicate;
- carbonates and hydrogencarbonates: These can be used as alkali metal-, earth alkali metal- or ammonium salts. Na-, Li- and Mg-carbonates and -hydrogen carbonate, especially sodium carbonate and/or sodium hydrogen carbonate are preferred;
- polyphosphates, such as pentanatriumtriphosphate.

### Useful as oligomeric and polymeric cobuilders are:

Oligomeric and polymeric carbonic acids, such as homopolymers of acrylic acid and aspartic acid, oligomaleic acid, copolymers of maleic acid and acrylic acid, methacrylic acid or C₂-C₂₂-olefines, e.g. isobutene or long chain α-olefines, vinyl-C₁-C₈-alkylether, vinylacetate, vinylpropionate, (meth)acryl acid ester of C₁-C₈-alcohols and styrene. Preferred are the homopolymers of acrylic acid and the copolymers of acrylic acid with maleic acid. The oligomeric and polymeric carbonic acids preferably are used as acids or as sodium salts.

Chelating agents are compounds, which can bind cations. They can be used to reduce water hardness and to precipitate heavy metals. Examples of complexing agents are: NTA, EDTA, MGDA, DTPA, DTPMP, IDS, HEDP, β-ADA, GLDA, citric acid, oxodisuccinic acid and butanetetracarbonic acid. The advantage of the use of these compounds lies in the fact that many compounds, which serve as cleaning agents, are more active in soft water. In addition to that scaling can be reduced or even be avoided. By using such compounds there is no need to dry a cleaned surface. This is an advantage in the work flow.

Useful anti greying agents are e.g. carboxymethylcellulose and graft polymers of vinyl acetate on polyethylene glycol.

Useful bleaching agents are e.g. adducts of hydrogenperoxide at inorganic salts, such as sodium perborate-monohydrate, sodium perborate-tetrahydrate and sodium carbonate-perhydrate, and percarbonic acids, such as phthalimidopercapronic acid.

As bleach activators compounds such as N,N,N',N'-tetraacetylethylendiamine (TAED), sodium-p-nonanoyloxybenzenesulfonate and N-methylmorpholiniumacetonitrilemethylsulfate are useful.

Useful enzymes are e.g. proteases, lipases, amylases, cellulases, mannanases, oxidases and peroxidases.

Useful as dye transfer inhibitors are e.g. homo-, co- and graft-polymers of 1-vinylpyrrolidone, 1-vinylimidazol or 4-vinylpyridine-N-oxide. Also homo- and copolymers of 4-vinylpyridin, which have been treated with chloro acetic acid are useful dye transfer inhibitors.

Biozides are compounds, which kill bacteria. An example of a biozide is glutaric aldehyde. The advantage of the use of biozides is that the spreading of pathogenic germs is counteracted.

Hydrotropes are compounds which enhance the solubility of the surfactant / the surfactants in the chemical composition. An example is: Cumolsulfonate.

Thickeners are compounds, which enhance the viscosity of the chemical composition. Non-limiting examples of thickeners are: polyacrylates and hydrophobically modified polyacrylates. The advantage of the use of thickeners is, that liquids having a higher viscosity have a longer residence time on the surface to be treated in the cases this surface is inclined or even vertical. This leads to an enhanced time of interaction.

The use of the inventive chemical compound or of the inventive chemical composition as washing and cleaning agent, surfactant, detergent, emulsifier, protection colloid in dispersions, dispergating agent, demulsifier, antifoaming agent, rheology modifier, melt-viscosity-reducer for polymers, fluidifier for glues and resins, fluidifier for dispersions, binder for glues and resins, binder for textiles, cross-linker in lack systems, solubilizing agent or carrier or encapsulation agent for aromatic and/or hydrophobic substances, melamine and melamine derivatives, dyes and biologically active substances, crop protection adjuvant in aqueous media and formulations, retanning agent for leather, hydrophobizating agent for leather and the use for the cosmetics industry forms another object of the present invention.

The use of the inventive chemical compound or of the inventive chemical composition as washing and cleaning agent, surfactant, detergent, demulsifier, antifoaming agent, rheology modifier, melt-viscosity-reducer for polymers, fluidifier for glues and resins, fluidifier for dispersions, solubilizing agent or carrier or encapsulation agent for aromatic and/or hydrophobic substances, melamine and melamine derivatives, dyes and biologically active substances, crop protection adjuvant in aqueous media and formulations forms a preferred object of the present invention.

The use of the inventive chemical compound or of the inventive chemical composition as washing and cleaning agent and as surfactant, forms a particularly preferred object of the present invention.

The invention will be described in more detail by the following non-limiting examples:

### Examples

In the following examples the OH number was determined according to DIN 53240-2. The viscosities given are dynamic viscosities, which were measured according to ISO 3219 using a cone plate viscosimeter. NMR was measured on an apparatus Bruker Avance 300 (300 MHz).

Example 1: 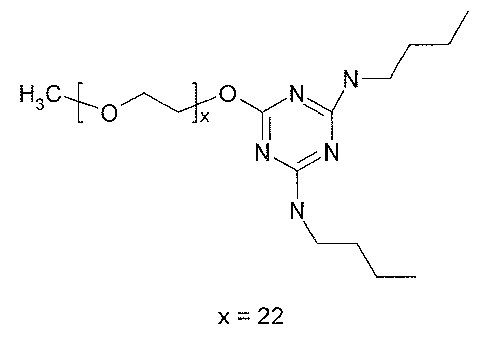 4.61 g (0.025 mol) of cyanuric chloride were solved in 50 ml pyridine in a flask. 25 g (0.025 mol) of PluriolA1000E® solved in 50 ml pyridine were slowly dropped into the flask at 5 °C. After 2 hours under stirring at 5 °C, the reaction mixture was slowly brought to room temperature. Subsequently 3.66 g (0.050 mol) of butyl amine were dropped at room temperature into the flask, before the reaction mixture was heated to 60 °C and stirred at this temperature for 2 hours. Finally the product was cooled to room temperature. 30.5 g of product were obtained as brown oil after evaporation of the solvent.

### Example 2 (theoretical):

By proceeding as in Example 1 but making the following amendments a product comprising two ho and one hi can be obtained: 50 g (0.050 mol) of PluriolA1000E® solved in 100 ml pyridine instead of 25 g (0.025 mol) of PluriolA1000E® solved in 50 ml pyridine and 1.83 g (0.025 mol) of butyl amine instead of 3.66 g (0.050 mol) of butyl amine are to be used.

### Example 3:

The properties of the reaction product from Example 1 were measured:

| | | | | **Average** |
|---|---|---|---|---|
| **c (mg/l)** | **c (g/l)** | **Surface tension (mN/m)** | **Surface tension (mN/m)** | **Surface tension (mN/m)** |
| 2,514 | 0,0025 | 70,9 | 70,5 | 70,7 |
| 5,648 | 0,0056 | 70,0 | 69,5 | 69,8 |
| 9,587 | 0,0096 | 69,3 | 68,9 | 69,1 |
| 14,487 | 0,0145 | 68,8 | 68,5 | 68,6 |
| 20,587 | 0,0206 | 68,4 | 68,0 | 68,2 |
| 28,164 | 0,0282 | 68,0 | 67,5 | 67,8 |
| 37,536 | 0,0375 | 67,6 | 66,9 | 67,3 |
| 49,086 | 0,0491 | 67,1 | 66,2 | 66,6 |
| 63,232 | 0,0632 | 66,3 | 65,2 | 65,7 |
| 80,460 | 0,0805 | 65,1 | 63,8 | 64,4 |
| 101,269 | 0,1013 | 63,4 | 61,6 | 62,5 |
| 126,164 | 0,1262 | 61,4 | 60,1 | 60,8 |
| 155,605 | 0,1556 | 59,8 | 58,8 | 59,3 |
| 189,969 | 0,1900 | 58,5 | 57,7 | 58,1 |
| 229,442 | 0,2294 | 57,4 | 56,7 | 57,0 |
| 273,984 | 0,2740 | 56,2 | 55,5 | 55,9 |
| 323,236 | 0,3232 | 54,6 | 54,0 | 54,3 |
| 376,489 | 0,3765 | 53,3 | 52,8 | 53,1 |
| 432,682 | 0,4327 | 52,0 | 51,6 | 51,8 |
| 490,493 | 0,4905 | 50,4 | 50,1 | 50,3 |
| 548,423 | 0,5484 | 48,9 | 48,8 | 48,9 |

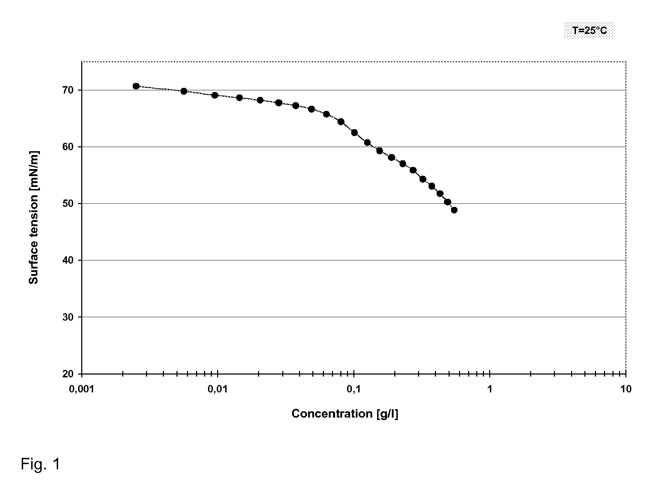

The result is displayed graphically in Fig. 1.

## Claims

1. Chemical compound according to formula (I) 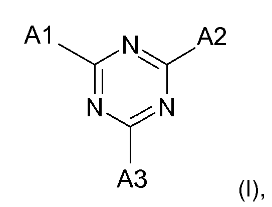 wherein
A1 and A2 independent from each other comprise a hydrophilic block (hi) and A3 comprises a hydrophobic block (ho) or wherein A1 and A2 independent from each other comprise a hydrophobic block (ho) and A3 comprises a hydrophilic block (hi).

2. Chemical compound according to claim 1, wherein hi is a non-ionic block.

3. Chemical compound according to one of claims 1 or 2, wherein the non-ionic block(s) is/are polyethyleneoxide.

4. Chemical compound according to one of claims 1 to 3, wherein ho is selected from the group consisting of amines having 1 to 100 C-atoms.

5. Chemical compound according to any one of claims 1 to 4, wherein A1 and A2 have the same structure.

6. Chemical composition comprising at least one chemical compound according to one of claims 1 to 5.

7. Chemical composition according to claim 6, wherein the at least one chemical compound according to one of claims 1 to 5 is present in an amount of 0.001 to 99.9 mass%.

8. Chemical composition according to claim 7, wherein the at least one chemical compound according to one of claims 1 to 5 is present in an amount of 0.1 to 99 mass%.

9. Chemical composition according to claim 8, wherein the at least one chemical compound according to one of claims 1 to 5 is present in an amount of 1 to 95 mass%.

10. Chemical composition according to one of claims 6 to 9, which composition contains at least one compound selected from the group consisting of surfactant, disinfectant, dye, acid, base, complexing agent, biocide, hydrotope, thickener, builder, cobuilder, enzyme, bleaching agent, bleach activator, bleaching catalyst, corrosion inhibitor, dye protection additive, dye transfer inhibitor, anti-greying agent, soil-release-polymer, fiber protection agent, silicon, bactericide, preserving agent, organic solvent, solubility adjustor, solubility enhancer and perfume.

11. Use of a chemical compound according to one of claims 1 to 5 or of a chemical composition according to one of claims 6 to 10 as washing and cleaning agent, detergent, emulsifier, protection colloid in dispersions, dispergating agent, demulsifier, antifoaming agent, rheology modifier, melt-viscosity-reducer for polymers, fluidifier for glues and resins, fluidifier for dispersions, binder for glues and resins, binder for textiles, cross-linker in lack systems, solubilizing agent or carrier or encapsulation agent for aromatic and/or hydrophobic substances, melamine and melamine derivatives, dyes and biologically active substances, crop protection adjuvant in aqueous media and formulations, retanning agent for leather, hydrophobizating agent for leather and use for the cosmetics industry.
